# EUROPEAN PATENT SPECIFICATION

(11) **EP 3 229 711 B1**
(45) Date of publication and mention of the grant of the patent: **11.09.2019**
(21) Application number: 15823543.2
(22) Date of filing: 04.12.2015
(51) Int. Cl.: A61B 17/22, A61B 17/221

(54) **TIPLESS RETRIEVAL DEVICE**
SPITZENLOSE RÜCKHOLVORRICHTUNG
DISPOSITIF DE RÉCUPÉRATION SANS POINTE

(30) Priority: 10.12.2014 US 201462089996 P
(43) Date of publication of application: 18.10.2017
(73) Proprietor: Boston Scientific Scimed, Inc., Maple Grove, MN 55311 (US)
(72) Inventor: TEAGUE, James, Spencer, IN 47460 (US); STEMLER, David, Coal City, IN 47427 (US)
(74) Representative: Pfenning, Meinig & Partner mbB
(86) International application number: PCT/US2015/063943
(87) International publication number: WO 2016/094229

(56) References cited:
- EP-A1- 0 941 036
- WO-A2-2014/008460
- US-A1- 2003 212 430
- US-A1- 2008 311 318
- US-A1- 2011 066 158
- US-B1- 9 101 383

## Description

### Cross-reference to Related Applications

This application claims the benefit of priority from U.S. Provisional Application No. 62/089,996, filed December 10, 2014.

### Technical Field

Various aspects of the present disclosure relate generally to retrieval devices. More specifically, the present disclosure relates to devices for retrieving objects within a patient.

### Background

Retrieval devices, such as baskets, are often used to remove organic material (e.g., blood clots, tissue, and biological concretions such as urinary, biliary, and pancreatic stones) and inorganic material (e.g., components of a medical device or other foreign matter), which may obstruct or otherwise be present within a patient's body cavities or passages. For example, concretions can develop in certain parts of the body, such as in the kidneys, pancreas, ureter, and gallbladder. Minimally invasive medical procedures are used to remove these concretions through natural orifices, or through an incision, such as during a percutaneous nephrolithotomy ("PNCL") procedure. Retrieval devices are also used in lithotripsy and ureteroscopy procedures to treat urinary calculi (e.g., kidney stones) in the ureter of a patient.

Current retrieval devices are often ill equipped to access tortuous anatomy and/or are prevented from retrieving objects that are either impacted against a wall of an anatomical lumen or positioned adjacent other anatomical structures. For example, current retrieval devices often include a distally extending tip portion which may interfere with and/or inhibit passage of the retrieval device about and behind such objects. The devices of the current disclosure may rectify some of the deficiencies described above.

For example, document EP 0 941 036 A1 describes a medical retrieval device, and related method, which uses a basket formed by one or more legs to retrieve material such as calculi. At least one of the legs has at least an inner and an outer surface. The outer surface is an atraumatic surface such as a curved surface. The atraumatic surface can include one or more radii. The inner surface can be flat such that the leg has a D-shaped cross section. Other shapes are possible for the inner surface including a pointed shape that enhances the basket's stone crushing or breaking ability. The inner surface, whatever its shape, can have a rough surface (e.g., serrated, etched, toothed, etc.) for further enhancing the basket's ability to capture stones and other calculi.

Document US 2003/212430 A1 describes designs, materials and manufacturing methods for medical retrieval devices. Some embodiments pertain to a medical retrieval device including a cage having internal migration barriers therein. Some embodiments pertain to a medical retrieval device including a cage having variable diameters along the length thereof. Several alternative medical retrieval devices constructions and/or designs including methods and techniques of construction are also disclosed.

Document US 2008/311318 A1 describes a multiwire unit having a plurality of wire sections which are formed in one piece from a tubular piece whose tubular jacket is subdivided in at least an axial subsection by a plurality of axial slots. The wire pieces remain interlinked at a front end section at a distance to a front tube face, and assume a bent functional state downstream of the wire linking area in a defined functional state of the multiwire unit. The front wire linking area, in the functional state of the multiwire unit, is shaped to an essentially tipless front end closure. For this purpose, the axial slots end at an appropriate small axial distance to the front tube face. The multiwire unit may be formed as, for example, a wire basket unit or a wire filter unit for medical instruments.

Document WO 2014/008460 A2 describes devices, systems, and methods for preventing, treating, and/or at least minimizing ischemia and/or reperfusion injury by restoring and/or modulating blood flow, particularly in the cerebral vasculature where blood vessels are narrow and tortuous. These devices, systems, and methods are intended to make it possible for a clinician to adequately and systematically restore blood flow to ischemic tissue while simultaneously modulating the blood flow to minimize reperfusion injury.

Document US 2011/066158 A1 describes a medical device, and related method, for manipulating material, such as calculi, within a patient's body, which includes a handle, a sheath, a retrieval basket, and a retainer coupled to the distal end of the retrieval basket. The retrieval basket includes a plurality of legs, each of which includes an intermediate portion located at the distal end of the retrieval basket. The retainer secures at least the intermediate portion of one of the plurality of legs to the intermediate portion of another of the plurality of legs.

### SUMMARY

The invention is described in the independent claim and preferred embodiments are listed in the dependent claims.

Examples of the present disclosure relate to, among other things, medical retrieval devices. Each of the examples disclosed herein may include one or more of the features described in connection with any of the other disclosed examples.

In one example, a medical retrieval device may include a longitudinally extending tubular member having a cut pattern. The pattern may include an x-shaped portion and a plurality of legs extending proximally of the x-shaped portion. At least one of the plurality of legs may have a varied cross-section along its length. The medical retrieval device may further include a longitudinally extending drive member operably coupled to the tubular member.

Examples of the medical retrieval device may additionally and/or alternatively include one or more of the following features: at least one of the plurality of legs may have a proximal portion with a semi-circular cross-sectional shape and a distal portion with a circular cross-section shape; the distal portion may be chemically etched; the plurality of legs and the x-shaped portion may be monolithically formed; the x-shaped portion may define a distal-most end of the medical retrieval device; a distal-most end of the medical retrieval device may be tipless; each leg of the plurality of legs may be cut into two distal leg portions; a distal leg portion of a first leg of the plurality of legs may be continuously formed with a distal leg portion of a second leg of the plurality of legs; the two distal leg portions of at least one leg of the plurality of legs may be coupled together; the tubular member may include shape memory material; the drive member may be configured to be received within the tubular member; the x-shaped portion may define a central opening; at least one strut may be coupled to at least one leg of the plurality of legs; the at least one strut may include a frame of struts coupled to each leg of the plurality of legs; and a handle assembly having an actuator may be configured to move the medical retrieval device between an extended state and a retracted state.

In another example, a method of forming a medical retrieval device may include cutting a pattern into a longitudinally extending tubular member. The pattern may include an x-shaped portion and a plurality of legs extending proximally of the x-shaped portion. The method may further include machining the pattern such that at least one of the plurality of legs has a varied cross-section along its length. Further, the method may include heat-treating the pattern to form a monolithic one-piece retrieval device.

Examples of the method may additionally and/or alternatively include one or more of the following features: wherein cutting a pattern may include laser cutting; wherein machining the pattern may include chemically etching the pattern such that at least one of the plurality of legs has a varied cross-section along its length; coupling a longitudinally extending drive member to the tubular member; and wherein coupling may include welding.

In another example, a method of forming a medical retrieval device may include cutting a longitudinally extending tubular member. The cutting may include forming a first cut line in the tubular member. The first cut line may oscillate back and forth between a first axial location along the tubular member and a distal-most end of the tubular member. The cutting may further include forming a second cut line in the tubular member. The second cut line may oscillate back and forth between a second axial location along the tubular member and the distal-most end of the tubular member. Additionally, forming the first cut line and forming the second cut line may define an x-shaped portion and a plurality of legs extending proximally of the x-shaped portion. The method may also include machining at least one of the plurality of legs such that is includes a varied cross-section along its length.

Examples of the method may additionally and/or alternatively include one or more of the following features: wherein cutting may include laser cutting; wherein machining may include chemically etching the at least one of the plurality of legs such that the at least one of the plurality of legs has a varied cross-section along its length; and after the cutting, heat-treating the tubular member to form a monolithic one-piece retrieval device.

It may be understood that both the foregoing general description and the following detailed description are exemplary and explanatory only and are not restrictive of the features, as claimed.

### BRIEF DESCRIPTION OF THE DRAWINGS

The accompanying drawings, which are incorporated in and constitute a part of this specification, illustrate exemplary features of the present disclosure and together with the description, serve to explain the principles of the disclosure.
FIG. 1 illustrates an exemplary retrieval device;
FIG. 2 illustrates a cross-sectional view of a portion of the exemplary retrieval device of FIG. 1;
FIG. 3 illustrates a cross-sectional view of another portion of the exemplary retrieval device of FIG. 1;
FIG. 4 illustrates a side-view of an exemplary end effector of the retrieval device of FIG. 1;
FIG. 5 illustrates an end-view of the exemplary end effector of FIG. 4;
FIG. 6 illustrates a side-view of a proximal portion of the exemplary end effector of FIG. 4;
FIG. 7 illustrates a cross-sectional shape of a proximal portion of a leg of the exemplary end effector of FIG. 4;
FIG. 8 illustrates a cross-sectional shape of a distal portion of a leg of the exemplary end effector of FIG. 4;
FIG. 9 illustrates an end-view of an additional exemplary end effector; and
FIGS 10 and 11 illustrate exemplary illustrate laser cutting patterns for forming the exemplary end effector of FIG. 4.

### DETAILED DESCRIPTION

### Overview

Examples of the present disclosure relate to a medical device for treating internal areas of a subject's body. The medical device may include a tipless retrieval basket.

### Detailed Examples

Reference will now be made in detail to examples of the present disclosure described above and illustrated in the accompanying drawings. Wherever possible, the same reference numbers will be used throughout the drawings to refer to the same or like parts.

The terms "proximal" and "distal" are used herein to refer to the relative positions of the components of an exemplary medical device. When used herein, "proximal" refers to a position relatively closer to the exterior of the body or closer to a medical professional using the medical device. In contrast, "distal" refers to a position relatively further away from the medical professional using the medical device, or closer to the interior of the body.

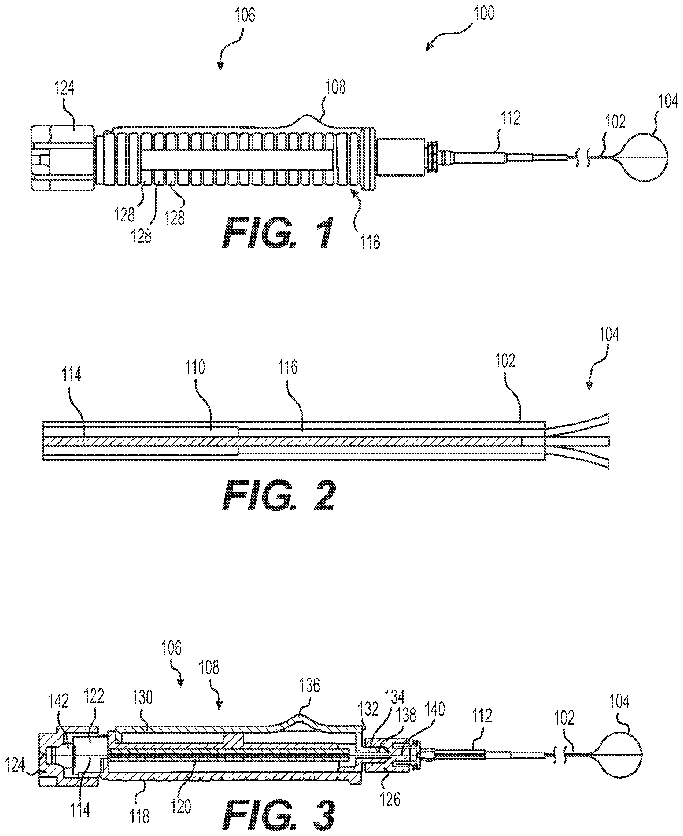

FIGS. 1-3 illustrate portions of a retrieval device 100. Retrieval device 100 may include a sheath 102 including a distal end and a proximal end. Retrieval device 100 may also include an end effector 104 at the distal end of sheath 102. At least a portion of end effector 104 may be movable relative to sheath 102 between an extended state and a retracted state. Retrieval device 100 may also include a handle assembly 106 at the proximal end of sheath 102. Handle assembly 106 may include an actuation member 108 for moving end effector 104 between the extended state and the retracted state, as will be described in further detail below. A strain relief member 112 may be coupled to handle assembly 106, and may extend at least partially over a proximal portion of sheath 102. Strain relief member 112 may help prevent sheath 102 from kinking at or near the distal end of handle assembly 106. As shown in FIG. 1, strain relief member 112 may have a varying cross-sectional shape and may include a polymer material, metal, or a combination of materials. Alternatively, retrieval device 100 may not include strain relief 112.

As shown in FIG. 2, sheath 102 may include a longitudinally-extending lumen 110. Sheath 102 may be, for example, a hollow tube. Sheath 102 may be made of a polymer material, metal, or a combination of materials. Retrieval device 100 may also include a drive member 114. Drive member 114 may extend through lumen 110 of sheath 102. Drive member 114 may be elongated, and may include, for example, a wire, braid, shaft, and/or any other suitable drive member configured to transfer translational and/or rotational forces from its proximal end to its distal end. As shown in FIG. 2, a distal end portion of drive member 114 may be coupled to end effector 104. For example, end effector 104 may include a tubular member 116, as will be described in further detail below, which may be configured to receive the distal end portion of drive member 114 therein. Drive member 114 may be secured within tubular member 116 via any appropriate connection mechanism such as, for example, laser welding, adhesives, and/or mechanical fasteners, etc. Alternatively, drive member 114 and end effector 104 may be monolithically formed so as to include a continuous one-piece structure. Additionally, in another alternative example, a proximal end of tubular member 116 may extend proximally along sheath 102 and be coupled to actuation member 108 within handle assembly 106. In such an arrangement, drive member 114 may be omitted and tubular member 116 may be used to move end effector 104 between the extended state and the retracted state.

As shown in FIG. 3, handle assembly 106 may include a grip 118 configured to movably receive the actuation member 108 therein. Grip 118 may include one or more baffles 128 (FIG. 1) configured to aid a medical professional with securely grasping grip 118. Any number and arrangement of baffles 128 may be disposed on grip 118. Grip 118 may be hollow and have a semi-circular cross-sectional shape. Actuation member 108 may be configured to be matingly received within grip 118. As shown in FIG. 2, actuation member 108 may include a longitudinally extending member 130 and a distal facing end 132 including a nipple 134. The longitudinally extending member 130 may include a raised surface 136 to ease manipulation of the medical device 100 by the medical professional. For example, the raised surface 136 may include a thumb or finger rest.

Nipple 134 may extend distally of distal facing end 132 and be configured to be coupled to a connector 126. For example, nipple 134 and connector 126 may be coupled via any appropriate means such as, for example, a screw fit connection and/or adhesive. Connector 126 may include a male luer fitting. As shown in FIG. 3, drive member 114 may extend proximally through a lumen 140 in connector 126, through a lumen 138 in nipple 134, through a support tube 120 along longitudinally extending member 130, and towards a retention member 122. Retention member 122 may be coupled to grip 118 and may form a vise 142, or any other suitable holding mechanism. When vise 142 closes, holding member drive member 114 may be fixedly coupled relative to grip 118. An end cap 124 may be placed onto proximal ends of holding member 122 and vise 142 to help close vise 142 around drive member 114. Vise 142 and/or retention member 122 may include an externally threaded portion (not shown), and end cap 124 may include complementary internal threading (not shown), such that end cap 124 may be screwed onto retention member 122 and vise 142.

In use, a medical professional may urge raised surface 136 distally relative to grip 118 so as to move end effector 104 between the extended state and the retracted state. For example, a medical professional may hold grip 118 within the palm of their hand with their thumb or finger on raised surface 136. In order to move end effector 104 from the extended state to the retracted state, the medical professional may push, slide, or advance raised surface 136 of actuation member 108 relative to grip 118. Due to the connection of sheath 102 to actuation member 108 via connector 126, moving raised surface 136 distally results in distal movement of sheath 102 over end effector 104. Upon advancement of sheath 102 over end effector 104, end effector 104 transitions (e.g., collapses, compresses, etc.) to its retracted state within lumen 110 of sheath 102.

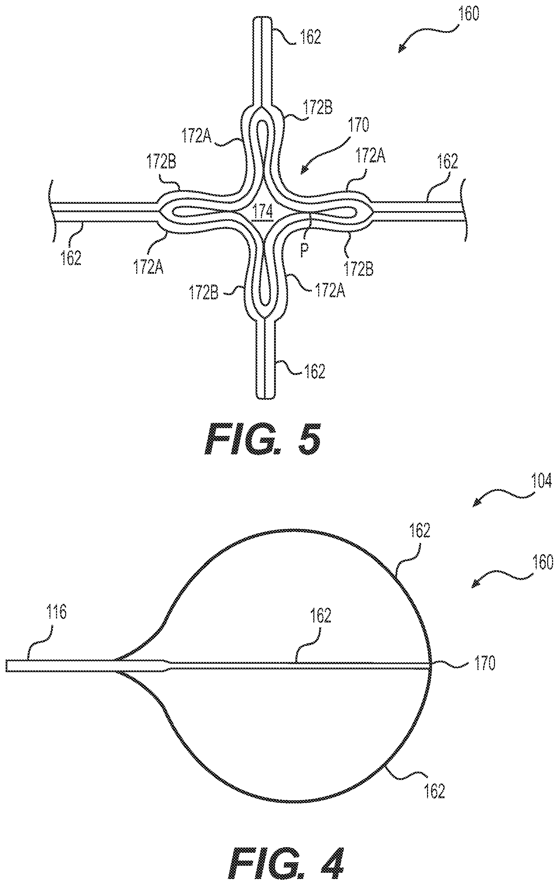

End effector 104 may include a basket 160, as shown in FIG. 4. Basket 160 may be formed from a tube. For example, a method of basket 160 formation involves starting with a hollow tube or cannula, such as tubular member 116. At least a portion of the tubular member 116 may then be cut (e.g., laser cut) with a cut pattern to produce one or more legs 162. While four legs 162 are shown in FIGS. 5, 6, and 8-10, it is understood that a greater or lesser number of legs 162 may be formed from tubular member 116. For axial basket 160 features (e.g., features extending substantially axially along a longitudinal axis of the underlying basket 160 structure) the cut pattern may include slots are cut lengthwise along the exterior of a wall of tubular member 116 substantially parallel to a longitudinal axis of tubular member 116. For helical, spiral, or other features that are not solely axial in direction (e.g., features that extend at an angle with respect to a longitudinal axis of tubular member 116), the cut pattern may include slots are cut with both an axial component and a radial component. After cutting, any one or more portions of the resultant legs 162 may be further manufactured and/or machined. For example, following cutting, one or more portions of one or more legs 162 may be chemically etched so as to alter a cross-sectional shape of the legs 162, as will be described in further detail below. Following cutting, optional chemical etching, and cleaning, the resulting form may be compressed lengthwise (e.g., expanded radially) to spread the legs 162 and manipulate the legs 162 into a desired shape. This shape may then be heat treated (e.g., annealed) into the material to form a desired basket 160 configuration. Accordingly, end effector 104 may be monolithically formed single piece of material.

According to examples of the present disclosure, the basket 160 configuration can be made at least partially of a shape-memory material. Shape-memory material is a material that can be formed into a particular shape, retain that shape during resting conditions (e.g., when the shaped material is not subject to external forces or when external forces applied to the shaped material are insufficient to substantially deform the shape), be deformed into a second shape when subjected to a sufficiently strong external force, and revert substantially back to the initial shape when external forces are no longer applied. Examples of shape memory materials include synthetic plastics, stainless steel, and superelastic metallic alloys of nickel/titanium (e.g., Nitinol), copper, cobalt, vanadium, chromium, iron, or the like. Alternative basket 160 materials include, but are not limited to, other metal alloys, powdered metals, ceramics, thermal plastic composites, ceramic composites, and polymers. Also, combinations of these and other materials can be used.

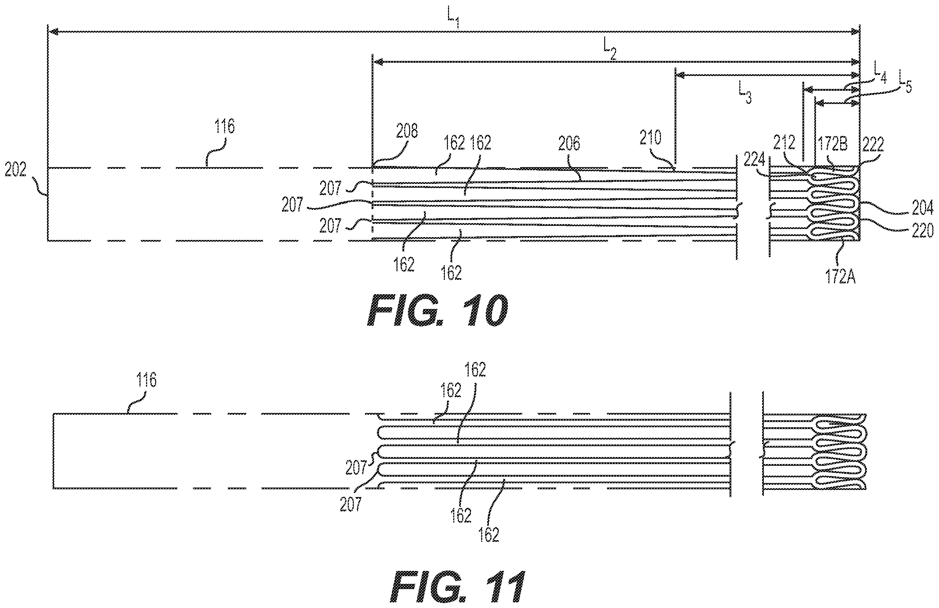

As shown in FIG. 4, a distal-most end 170 of basket 160 may be flat. In other words, basket 160 may be "tipless." That is, basket 160 may be manufactured to not include any distally protruding tip or material distal of legs 162. In other words, the basket 160 may be manufactured to be flat in a direction perpendicular to a longitudinal axis of the basket 160. For example, as shown in FIG. 5, distal-most end 170 of basket 160 may have a cut pattern that is cut from tubular member 116 in the form of a cross or x-shape. To form the cut pattern with a cross or x-shape, a distal portion of each leg 162 may be divided (e.g., cut) into two leg portions 172a and 172b. As shown in FIG. 5, adjacent distal leg portions 172a, 172b, may be continuously formed. That is, distal leg portion 172a of a first leg 162 may be continuously formed with an adjacent distal leg portion 172b of an adjacent second leg 162. Optionally, the two leg portions 172a and 172b of each leg 162 may be joined together therebetween, such as at point P. Leg portions 172a and 172b may be joined through any appropriate means such as, for example, welding, adhesives, and the like. Joining leg portions 172a and 172b together may increase the strength, durability, and/or stability of end effector 104. Alternatively, leg portions 172a and 172b may not be joined together, thereby providing increased flexibility to end effector 104. Additionally, a central distal opening 174 may be formed between the adjacent distal leg portions 172a, 172b. The manner of cutting basket 160 from tubular member 116 will be described in further detail below in regard to FIGS. 10 and 11.

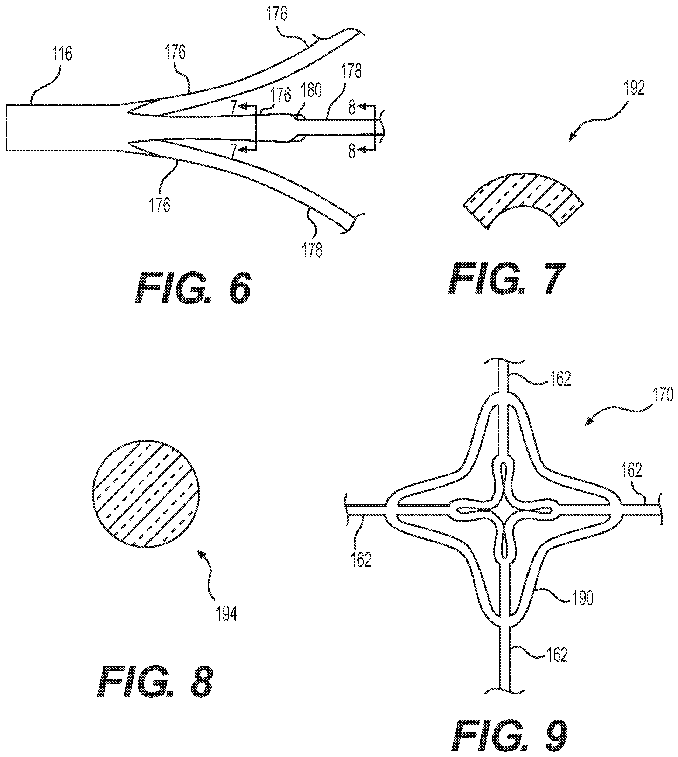

As shown in FIG. 6, a proximal portion of each leg may include a varied cross-section. For example, a proximal-most portion 176 of each leg 162 may have a first cross-sectional shape, while a distal portion 178 of each leg 162 may have a second cross-sectional shape. For example, in some examples, as shown in FIG. 7, the first cross-sectional shape may be a semicircular shape 192 while the second cross-sectional shape, as shown in FIG. 8, may be a circular shape 194. For instance, each leg 162 may include a transition portion 180 at which the leg transitions from the first cross-sectional shape to the second cross-sectional shape. The transition portion 180 may include a tapered region of each leg 162. In other words, the proximal-most portion 176 may include a first cross-sectional area, and the distal portion 178 may include a second cross-sectional area, while the transition portion 180 may have a transition cross-sectional area. The second cross-sectional area may be smaller than the first cross-sectional area. Additionally, the transition cross-sectional area may have a size between the first cross-sectional area and the second cross-sectional area. The transition portion 180 may connect the proximal-most portion 176 and the distal portion 178 of each leg 162 and define a sloping or arcuate portion of each leg 162. The degree of slope or curvature of transition portion 180 may be any appropriate degree. For example, as shown in FIG. 6, transition portion 180 may smoothly and gradually transition from first cross-sectional area to the second cross-sectional area. In such arrangements, for example, the degree of sloping or curvature may be between about 5° and about 85°. Accordingly, the degree of sloping or curvature may be between about 45° and about 60°. In other examples, transition portion 180 may sharply and/or abruptly transition from first cross-sectional area to the second cross-sectional area. In such arrangements, for example, the degree of sloping or curvature may again be between about 5° and about 85°. Accordingly, the degree of sloping or curvature may be between about 45° and about 60°.

Varied cross-sectional area of one or more legs 162 of basket 160 may assist end effector 104 in transitioning between the retracted state and the expanded state and manipulating the end effector 104 during use. For instance, in examples in which proximal-most portion 176 of each leg 162 includes a semi-circular cross-sectional shape 192 as shown in FIG. 7, proximal-most portion 176 may provide greater dilatation and/or expansion force than distal portion 178 of each leg 162. That is, upon transitioning between the retracted state and the expanded state of end effector 104, the larger cross-sectional area of proximal-most portion 176 may assist basket 160 to expand. Additionally, in examples in which the distal portion 178 includes a circular cross-sectional shape 194 as shown in FIG. 8, end effector 104 may have increased flexibility and maneuverability near distal portion 178. That is, the smaller cross-sectional area of the distal portion 178 may allow the distal portion 178 of basket 160 to be more readily bent or positioned about material which may obstruct or otherwise be present within a patient's body cavities or passages.

Optionally, as shown in FIG. 9, distal-most end 170 of basket 160 may further include one or more struts 190. For example, in some arrangements, one or more struts 190 may be positioned between each leg 162. Struts 190 may be coupled to the legs 162 via any appropriate means such as, for example, welding and/or adhesives. Further, it is understood that struts 190 may include a single continuously formed frame of struts, and/or a plurality of individual struts 190. Struts 190 may increase the strength, durability, and/or stability of end effector 104.

FIGS. 10 and 11 illustrate laser cutting patterns for cutting tubular member 116 to form basket 160. The patterns shown in FIGS. 10 and 11 depict tubular member 116 as if it were laid out flat. As shown in FIG. 10, tubular member 116 may have a length L₁ extending between a proximal-most end 202 and a distal-most end 204. Length L₁ may be about 1.2441 inches (31.6 mm). As used herein, the terms "about," "substantially," and "approximately," may indicate a range of values within +/- 5% of a stated value. As shown in FIGS. 10 and 11, tubular member 116 may be laser cut to form the patterns shown. That is, a single continuous cut line 206 may be formed through a wall of tubular member 116. For example, a cut line 206 may be started at a first axial location 208 along a side wall of tubular member 116. Location 208 may extend a length L₂ away from distal-most end 204 of tubular member. Length L₂ may be about 1.0079 inches (25.6 mm). Cut line 206 may extend from location 208 towards the distal-most end 204 and back in a substantially zig-zag or oscillating pattern thereby defining legs 162. The connection 207 between the proximal end of each leg 162 may be rounded such as at a diameter of about 0.0020 inches (0.051 mm) as shown in FIG. 10 or about 0.0084 inches (0.213 mm) as shown in FIG. 11. Alternatively, the connection 207 between legs 162 may not be rounded.

As shown in FIG. 10, at least a portion of cut line 206 may be angled such that legs 162 are tapered along their length. While the embodiment of FIG. 6 illustrates a rather sharp, short, and/or quick transition between the proximal-most portion 176 and the distal portion 178 of each leg 162, the example shown in FIG. 10 illustrates an elongated, extended, and/or slow transition. That is, for example and as shown in FIG. 10, cut line 206 may taper at an angle from location 208 to a second axial location 210. Location 210 may extend a length L₃ away from distal-most end 204 of tubular member. Length L₃ may be about 0.7879 inches (20.0 mm). The angle of tapering may be about 1°. The cut line 206 may then extend substantially straight (e.g., not angled or tapered) from point 210 towards the distal-most end 204 of tubular member 116. Alternatively, as shown in FIG. 11, cut line 206 may not be angled such that legs 162 are not tapered along their length.

As cut line 206 approaches distal-most end 204 of tubular member 116, cut line 206 may be curved or otherwise rounded at third axial location 212 to define proximal facing surfaces of distal leg portions 172a, 172b of each leg 162. Cut line 206 may be rounded at location 212 with a radius of about 0.0050 inches (0.127 mm). Location 212 may extend a length L₄ away from distal-most end 204 of tubular member 116. Length L₄ may be about 0.0416 inches (1.06 mm). In addition to cut line 206, a second cut line 220 may be laser cut through a wall of tubular member 116. For example, cut line 220 may be started at fourth axial location 222 along distal-most end 204 of tubular member 116 and may extend proximally towards fifth axial location 224 to thereby define a distal facing surface of each of distal leg portions 172a, 172b. Location 224 may extend a length L₅ away from distal-most end 204 of tubular member 116. Length L₅ may be about 0.0326 inches (0.83 mm). In this manner, tubular member 116 may be cut so as to define basket 160. It is understood that each basket may be cleaned (e.g., electro-polished) and/or chemically etched following completion of laser cutting. It is also understood that cut lines 206 and 220 may extend entirely through the thickness of the tubular member 116 and that tubular member 116 may be rotated during cutting to facilitate cutting therethrough.

In use, a medical professional may deliver retrieval device 100 to a location within the subject's body via any appropriate means. For example, an insertion device (not shown) may be used to access and view internal areas of a subject's body. Such an insertion device may include any device configured to deliver medical instruments, such as, for example, biopsy forceps, graspers, baskets, snares, probes, scissors, retrieval devices, lasers, and/or other tools, into a subject's body. The insertion device may be inserted into a variety of body openings, lumens, and/or cavities. For example, the insertion device may be inserted into any portion of a urinary tract, such as a ureter, a gastrointestinal lumen, such as an esophagus, a vascular lumen, and/or an airway. According to aspects of the present disclosure, the insertion device may be a ureteroscope. In some contemplated examples, the insertion device may be a sterile, single-use, and disposable ureteroscope. Alternatively, the insertion device may be a multiple-use, non-disposable ureteroscope. Other types of devices, however, may be substituted for the ureteroscope, including, as examples, an endoscope, a hysteroscope, a uteroscope, a bronchoscope, a cystoscope, and similar devices. Such devices may be single-use and disposable, or multiple-use and non-disposable.

Once positioned within the subject's body, the medical professional may move end effector 104 between the extended state and the retracted state. In the extended state, end effector 104 may be caused to capture, retain, remove, or otherwise collect material (e.g., blood clots, tissue, and biological concretions such as urinary, biliary, pancreatic stones, components of a medical device and/or other foreign matter), which may obstruct or otherwise be present within a patient's body cavities or passages. By virtue of the tipless design of end effector 104, end effector 104 may access tortuous anatomy and/or may retrieve objects that are either impacted against a wall of an anatomical lumen or positioned adjacent other anatomical structures. For example, the legs 162 of the tipless end effector 104 may be passed about and behind such objects for safe and efficient removal.

While principles of the present disclosure are described herein with reference to illustrative examples for particular applications, it should be understood that the disclosure is not limited thereto. Those having ordinary skill in the art and access to the teachings provided herein will recognize additional modifications, applications, embodiments, in accordance with the appended claims. Accordingly, the claimed features are not to be considered as limited by the foregoing description.

## Claims

1. A medical retrieval device (100), comprising:
a longitudinally extending tubular member (116) having a cut pattern, wherein the pattern includes an x-shaped portion and a plurality of legs (162) extending proximally of the x-shaped portion, wherein at least one of the plurality of legs (162) has a varied cross-section along its length; and
a longitudinally extending drive member (114) operably coupled to the tubular member (116), wherein the at least one of the plurality of legs (162) has a proximal portion with a semi-circular cross-sectional shape (192) and a distal portion (178) with a circular cross-section shape (194).

2. The medical retrieval device (100) of claim 1, wherein the distal portion (178) is chemically etched.

3. The medical retrieval device (100) of any one of the preceding claims, wherein the plurality of legs (162) and the x-shaped portion are monolithically formed.

4. The medical retrieval device (100) of any one of the preceding claims, wherein the x-shaped portion defines a distal-most end of the medical retrieval device (100).

5. The medical retrieval device (100) of any one of the preceding claims, wherein a distal-most end of the medical retrieval device (100) is tipless.

6. The medical retrieval device (100) of any one of the preceding claims, wherein each leg (162) of the plurality of legs (162) is cut into two distal leg portions (172a, 172b).

7. The medical retrieval device (100) of claim 6, wherein a distal leg portion (172) of a first leg (162)of the plurality of legs (162) is continuously formed with a distal leg portion (172b) of a second leg (162) of the plurality of legs (162).

8. The medical retrieval device (100) of claim 6, wherein the two distal leg portions (172a, 172b) of at least one leg (162) of the plurality of legs (162) are coupled together.

9. The medical retrieval device (100) of any one of the preceding claims, wherein the tubular member (116) comprises shape memory material.

10. The medical retrieval device (100) of any one of the preceding claims, wherein the drive member (114) is configured to be received within the tubular member (116).

11. The medical retrieval device (100) of any one of the preceding claims, wherein the x-shaped portion defines a central opening (174).

12. The medical retrieval device (100) of any one of the preceding claims, further including:
at least one strut (190) coupled to at least one leg (162) of the plurality of legs (162).

13. The medical retrieval device (100) of claim 12, wherein the at least one strut (190) comprises a frame of struts coupled to each leg (162) of the plurality of legs (162).

14. The medical retrieval device (100) of any one of the preceding claims, further including:
a handle assembly (106) having an actuator configured to move the medical retrieval device (100) between an extended state and a retracted state.

## Patentansprüche

1. Medizinische Rückholvorrichtung (100), welche aufweist:
ein sich in Längsrichtung erstreckendes rohrförmiges Teil (116) mit einem geschnittenen Muster, bei der das Muster einen x-förmigen Bereich und mehrere Beine (162), die sich proximal von dem x-förmigen Bereich erstrecken, enthält, wobei zumindest eines der mehreren Beine (162) einen variierenden Querschnitt entlang seiner Länge hat; und
ein sich in Längsrichtung erstreckendes Antriebsteil (114), welches betriebsmäßig mit dem rohrförmigen Teil (116) gekoppelt ist, wobei das zumindest eine der mehreren Beine (162) einen proximalen Bereich mit einer halbkreisförmigen Querschnittsform (192) und einem distalen Bereich (178) mit einer kreisförmigen Querschnittsform (194) hat.

2. Medizinische Rückholvorrichtung (100) nach Anspruch 1, bei der der distale Bereich (178) chemisch geätzt ist.

3. Medizinische Rückholvorrichtung (100) nach einem der vorhergehenden Ansprüche, bei der die mehreren Beine (162) und der x-förmige Bereich einstückig gebildet sind.

4. Medizinische Rückholvorrichtung (100) nach einem der vorhergehenden Ansprüche, bei der der x-förmige Bereich ein distal-äußerstes Ende der medizinischen Rückholvorrichtung (100) definiert.

5. Medizinische Rückholvorrichtung (100) nach einem der vorhergehenden Ansprüche, bei der ein distal-äußerstes Ende der medizinischen Rückholvorrichtung (100) spitzenlos ist.

6. Medizinische Rückholvorrichtung (100) nach einem der vorhergehenden Ansprüche, bei der jedes Bein (162) der mehreren Beine (162) in zwei distale Beinbereiche (172a, 172b) geschnitten ist.

7. Medizinische Rückholvorrichtung (100) nach Anspruch 6, bei der ein distaler Beinbereich (172) eines ersten Beins (162) der mehreren Beine (162) kontinuierlich mit einem distalen Beinbereich (172b) eines zweiten Beins (162) der mehreren Beine (162) gebildet ist.

8. Medizinische Rückholvorrichtung (100) nach Anspruch 6, bei der die beiden distalen Beinbereiche (172a, 172b) des zumindest einen Beins (162) der mehreren Beine (162) miteinander gekoppelt sind.

9. Medizinische Rückholvorrichtung (100) nach einem der vorhergehenden Ansprüche, bei der das rohrförmige Teil (116) Formspeichermaterial aufweist.

10. Medizinische Rückholvorrichtung (100) nach einem der vorhergehenden Ansprüche, bei der das Antriebsteil (114) konfiguriert ist, innerhalb des rohrförmigen Teils (116) aufgenommen zu werden.

11. Medizinische Rückholvorrichtung (100) nach einem der vorhergehenden Ansprüche, bei der der x-förmige Bereich eine mittlere Öffnung (174) definiert.

12. Medizinische Rückholvorrichtung (100) nach einem der vorhergehenden Ansprüche, weiterhin enthaltend:
zumindest eine Strebe (190), die mit zumindest einem Bein (162) der mehreren Beine (162) gekoppelt ist.

13. Medizinische Rückholvorrichtung (100) nach Anspruch 12, bei der die zumindest eine Strebe (190) einen Rahmen aus Streben aufweist, der mit jedem Bein (162) der mehreren Beine (162) gekoppelt ist.

14. Medizinische Rückholvorrichtung (100) nach einem der vorhergehenden Ansprüche, weiterhin enthaltend:
eine Handgriffanordnung (106) mit einem Aktuator, der konfiguriert ist, die medizinische Rückholvorrichtung (100) zwischen einem ausgefahrenen Zustand und einem zurückgezogenen Zustand zu bewegen.

## Revendications

1. Dispositif médical de récupération (100), comprenant :
un élément tubulaire s'étendant longitudinalement (116) qui présente un motif découpé, dans lequel le motif découpé inclut une section en forme de x et une pluralité de jambages (162) qui s'étendent de façon proximale par rapport à la section en forme de x, dans lequel au moins l'un de la pluralité de jambages (162) présente une section en coupe transversale variable suivant sa longueur ; et
un élément de pilotage s'étendant longitudinalement (114) qui est couplé de manière fonctionnelle à l'élément tubulaire (116), dans lequel l'au moins un de la pluralité de jambages (162) comporte une section proximale qui présente une forme en coupe transversale semi-circulaire (192) et une section distale (178) qui présente une forme en coupe transversale circulaire (194).

2. Dispositif médical de récupération (100) selon la revendication 1, dans lequel la section distale (178) est gravée chimiquement.

3. Dispositif médical de récupération (100) selon l'une quelconque des revendications précédentes, dans lequel la pluralité de jambages (162) et la section en forme de x sont formés de façon monolithique.

4. Dispositif médical de récupération (100) selon l'une quelconque des revendications précédentes, dans lequel la section en forme de x définit une extrémité la plus distale du dispositif médical de récupération (100).

5. Dispositif médical de récupération (100) selon l'une quelconque des revendications précédentes, dans lequel une extrémité la plus distale du dispositif médical de récupération (100) est sans pointe.

6. Dispositif médical de récupération (100) selon l'une quelconque des revendications précédentes, dans lequel chaque jambage (162) de la pluralité de jambages (162) est découpé selon deux sections de jambage distales (172a, 172b).

7. Dispositif médical de récupération (100) selon la revendication 6, dans lequel une section de jambage distale (172a) d'un premier jambage (162) de la pluralité de jambages (162) est formée en continu avec une section de jambage distale (172b) d'un second jambage (162) de la pluralité de jambages (162).

8. Dispositif médical de récupération (100) selon la revendication 6, dans lequel les deux sections de jambage distales (172a, 172b) d'au moins un jambage (162) de la pluralité de jambages (162) sont couplées ensemble.

9. Dispositif médical de récupération (100) selon l'une quelconque des revendications précédentes, dans lequel l'élément tubulaire (116) comprend un matériau à mémoire de forme.

10. Dispositif médical de récupération (100) selon l'une quelconque des revendications précédentes, dans lequel l'élément de pilotage (114) est configuré de manière à ce qu'il soit reçu à l'intérieur de l'élément tubulaire (116).

11. Dispositif médical de récupération (100) selon l'une quelconque des revendications précédentes, dans lequel la section en forme de x définit une ouverture centrale (174).

12. Dispositif médical de récupération (100) selon l'une quelconque des revendications précédentes, incluant en outre :
au moins une entretoise (190) qui est couplée à au moins un jambage (162) de la pluralité de jambages (162).

13. Dispositif médical de récupération (100) selon la revendication 12, dans lequel l'au moins une entretoise (190) comprend une structure d'entretoises qui sont couplées à chaque jambage (162) de la pluralité de jambages (162).

14. Dispositif médical de récupération (100) selon l'une quelconque des revendications précédentes, incluant en outre :
un assemblage de manche ou poignée (106) qui comporte un actionneur qui est configuré de manière à ce qu'il déplace le dispositif médical de récupération (100) entre un état étendu et un état rétracté.
